# EUROPÄISCHE PATENTSCHRIFT

(11) **EP 2 014 262 B1**
(45) Veröffentlichungstag und Bekanntmachung des Hinweises auf die Patenterteilung: **03.07.2019**
(21) Anmeldenummer: 08155965.0
(22) Anmeldetag: 09.05.2008
(51) Int. Cl.: A61F 2/42

(54) **Gelenkteilprothese mit spreizbarem Schaft**
Joint prosthetic with expandable shaft
Prothèse articulaire dotée d'une tige extensible

(30) Priorität: 09.07.2007 DE 202007009619 U
(43) Veröffentlichungstag der Anmeldung: 14.01.2009
(73) Patentinhaber: Zrinski AG, 78573 Wurmlingen (DE)
(72) Erfinder: Lindner, Nicola, 78573 Wurmlingen (DE); Reitzig, Cliff-Georg, 78604 Rietheim-Weilheim (DE); Eckhof, Stephan, 78604 Rietheim-Weilheim (DE); Feldhaus, Thomas, 78532 Tuttlingen (DE)
(74) Vertreter: Heisel, Wolfgang

(56) Entgegenhaltungen:
- WO-A-03/099171
- WO-A-2005/030087
- WO-A-2006/074414
- DE-B1- 2 338 136
- DE-U1- 29 514 169
- FR-A- 2 653 660
- US-A- 3 805 302
- US-A1- 2003 004 576
- US-A1- 2003 040 805
- US-B1- 6 251 141

## Beschreibung

### Technisches Gebiet

Die Erfindung bezieht sich auf eine Gelenkteilprothese, insbesondere für ein Fingergelenk, bestehend entweder aus einer proximalen oder einer distalen Komponente sowie einem von dieser Komponente wegweisenden, in einem Knochen zu lagernden Schaft, der zumindest zum Teil als Spreizhülse ausgebildet ist und eine durchgehende Bohrung aufweist, innerhalb der ein in dieser verschiebbarer Spreizkörper vorgesehen ist.

### Stand der Technik

Künstliche Fingergelenke bestehen im Wesentlichen aus zwei Elementen, nämlich einer proximalen und einer distalen Komponente. Eine Komponente weist einen konvexen Gelenkkopf auf, die mit dem anderen Teil, das eine konkave Gelenkschale zeigt, zusammenwirkt.

Künstliche Fingergelenke werden zwischen dem Mittelhandknochen und dem Fingerknochen oder zwischen einzelnen Fingerknochen eingesetzt. Es ist auch bekannt, dass ausschließlich das funktional defekte Fingergelenk ausgetauscht werden kann, so dass nur eine künstliche Komponente vorhanden ist.

Solche Gelenke müssen dann eingesetzt werden, wenn degenerative Gelenkkrankheiten, wie Osteoarthritis, posttraumatische Arthritis oder Rheumatuid-Arthritis der jeweiligen Gelenke vorliegen. Eine Alternative ist, die Fingergelenke zu versteifen. Eine andere Alternative, die die Mobilität der einzelnen Fingerglieder erhält, ist der totale Gelenkersatz.

Aus dem Stand der Technik, insbesondere aus der EP 1 203 569 A (FINSBURY (DEVELOPMENT) LIMITED LEATHERHEAD) 03.11.2000 sind Fingergelenkimplantate in der Ausführung sogenannter PIP-Schaftimplantate bekannt. Sie weisen eine proximale oder distale Komponente auf, die entsprechend zusammenwirken und jeweils einen von diesen Komponenten wegweisenden Schaft aufweisen. Der Schaft ist in der Regel konisch ausgebildet und wird in das Knochenmark eines Knochens eingeführt. Um die Einführung zu ermöglichen, wird mit einem Räumwerkzeug das Knocheninnere teilweise ausgeräumt und mit einem hammerartigen Instrument das Implantat in den Knochen eingeschlagen. Die Fixierung erfolgt derart, dass der Durchmesser der ausgeräumten Bohrung kleiner ist als der Außendurchmesser des Schaftes, so dass eine Art Presspassung zwischen dem Schaft und dem Inneren des Knochens entsteht.

Das Implantat besteht selbst aus einem Material mit einem knochenähnlichen Elastizitätsmodul. Dieses knochenähnliche Elastizitätsmodul vermeidet das sogenannten Stress-Shilding und fördert somit den Knochenaufbau. Dadurch wird zusätzlich die Fixierung des Implantats im Markraum des Knochens verbessert.

Ferner ist aus der US 3 805 302 A (MATHYS R) eine Fingergelenkprothese bekannt, die je Komponente des Gelenks einen in einen Fingerknochen einsetzbaren Schaft besitzt, der zumindest teilweise als eine Spreizhülse ausgebildet ist und der eine durchgehende Bohrung aufweist, damit ein von außen in diese Bohrung einsteckbarer Spreizstift, nach dem Einsetzen des Schaftes in eine vorbereitete Bohrung eines Fingerknochens, von der Gelenkseite her mittels eins Schraubenziehers in die Spreizhülse hineingeschraubt und dadurch diese gespreizt werden kann.

Die hierbei verwendete Spreizhülse besitzt auf ihrem Umfang sägezahnförmige Rippen und soll mit diesen Rippen durch den Spreizvorgang satt in der Bohrung des jeweiligen Knochens zum Anliegen kommen, was quasi als eine Art Sicherung gegen eine mögliche Lockerung der Spreizhülse gedacht ist.

### Nachteile des Standes der Technik

Die gemäss dem Stand der Technik angegebenen Prothesen weisen jedoch Nachteile auf, wie Implantatlockerung aufgrund mangelnder Fixierung und mangelnder Verbindung mit dem Knochen, wodurch hoher Verschleiß und entsprechender Abrieb entstehen kann. Die Folge ist, dass sich die schaftähnliche Konstruktion innerhalb des Markraumes lockern kann, so dass ein einmal fixiertes Implantat nicht mehr funktionsgerecht vorliegt. Hinsichtlich der Verwendung von Spreizhülsen ist es auch möglich, dass sich der hierbei eingesetzte Spreizstift bei nicht ausreichender Sicherung zurückdrehen kann, wodurch sich dann die Spreizung und somit die Fixierung des jeweiligen Schaftes lockert. Die Folgen davon sind wiederum Schmerzen, die auch durch Migration oder sogar Brüche hervorgerufen werden können.

### Aufgabe der Erfindung

Daher ist es Aufgabe der Erfindung, eine Gelenkteilprothese, insbesondere für Fingergelenke derart weiterzubilden, dass die Wahrscheinlichkeit einer Lockerung des Implantats im Gegensatz zum Stand der Technik verringert wird.

### Lösung der Aufgabe

Diese Aufgabe wird erfindungsgemäß dadurch gelöst, dass bei der Verwendung einer Spreizung zumindest jeweils im Anfangsbereich der durchgehenden Bohrung des mit einer Spreizhülse versehenen Schaftes und des Spreizkörpers ein Gewinde vorgesehen ist, welche derart zusammenwirken, dass nach einem Eindrehen des Spreizkörpers in die Bohrung, neben dem Zusammenwirken der beiden Gewinde, teilweise das Gewinde des Spreizkörpers zur Erzeugung einer Haftreibung zusätzlich in die Bohrung des Schaftes eingeschnitten wird.

### Vorteile der Erfindung

Ein Vorteil der Erfindung ist es, dass aufgrund der erfindungsgemäßen Ausgestaltung der Gelenkteilprothese es nicht notwendig ist, den jeweiligen Prothesenschaft durch eine schlagende bzw. hämmernde Bearbeitung des Markraums eines Knochens, in diesem zu platzieren. Demgegenüber ist die erfindungsgemäße Gelenkteilprothese einfach und effizient fixierbar.

Ein besonderer Vorteil der Erfindung ist es, dass ohne hohen Kraftaufwand die Gelenkteilprothese mit ihrem Schaft in den Markraum eines Knochens eingefügt und in einem weiteren Schritt dort mittels der vorgesehenen Spreizhülse fixiert werden kann. Diese zumindest teilweise über den Schaft sich erstreckende Spreizhülse wird durch einen innerhalb der Spreizhülse vorgesehenen, in Längsrichtung des Schafts verschiebbaren Spreizkörper aktiviert. Hierfür ist nach Maßgabe der Erfindung, die Bohrung des Schaftes im Bereich der Spreizhülse an deren freiem Ende und das freie Ende des Spreizkörpers jeweils breiter ausgebildet.

Durch eine derart verbreiterte Ausbildung wird bei einem Spreizvorgang die Spreizhülse mit ihrer Außenfläche gegen die Innenwandung der jeweiligen Knochenbohrung gepresst, und zwar indem der Spreizkörper derart in die Spreizhülse bzw. den Schaft eingeschraubt wird, dass sich nach der eigentlichen Gewindepaarung zwischen dem Schaft und dem Spreizkörper das Gewinde des Spreizkörpers sich noch zusätzlich in den Schaft wie eine Selbstschneideschraube in diesen einschneidet, sodass praktisch aufgrund der hierbei auftretenden Haftreibung der Spreizprozess quasi eingefroren und dadurch eine mögliche spätere Lockerung einer derartigen Befestigung verhindert wird.

Hierzu ist es zweckmäßig, den Spreizkörper derart auszubilden, dass er auf seinem von der Komponente wegweisenden Seite in seinem Durchmesser größer ausgebildet ist als im übrigen Teil des Schaftes, so dass durch Eindrehen bzw. Ausdrehen, beispielsweise mit einem Schraubendreher, des Spreizkörpers die Spreizhülsen auseinander gespreizt werden und sich an der Wandung des Markraumes abstützen beziehungsweise dort verankern.

Damit überhaupt eine Spreizung in dieser Form möglich ist, kann vorteilhafterweise vorgesehen sein, dass die Spreizhülse derart ausgebildet ist, dass ein herkömmlicher Schaft kanuliert ausgebildet ist und an seinem freien Ende entsprechende Schlitze vorgesehen sind, die sich ebenfalls in Längserstreckung des Schaftes ausrichten. Der Spreizkörper selbst ist innerhalb des Schaftes in einer durchgehenden kanulierten Bohrung gelagert und vorzugsweise über ein innerhalb des Schaftes angeordnetes Gewinde auch in und gegen den Uhrzeigersinn drehbar. In eine Richtung drehbar bedeutet, dass die Spreizung ausgeführt wird, wohingegen in die andere Richtung die Spreizung aufgehoben wird.

Ferner kann bei einer vorteilhaften Ausführungsform vorgesehen sein, ein Hilfsmittel als Drehwerkzeug über eine Sollbruchstelle mit dem Spreizkörper zunächst einstückig auszubilden. Durch ein Drehen des Spreizkörpers mittels des Hilfsmittels als Drehwerkzeug wird die entsprechende Spreizung des Schaftes beziehungsweise der Spreizhülse ausgeübt und bei Erreichen eines bestimmten Drehmoments bricht die Verbindung zwischen der Spreizhülse und dem Drehwerkzeug ab, da an einer definierten Stelle die entsprechende Sollbruchstelle vorgesehen ist. Die Materialeigenschaften sind dabei derart gewählt, dass eine nachteilige Spanbildung verhindert wird.

Auch ist es zweckmäßig, dass sich der Spreizkörper aus der durchgehenden Bohrung des freien Endes des Schaftes zumindest geringfügig heraus erstreckt und so den offenen Bereich des Gelenkteilimplantats abdeckt. Dadurch wird vorteilhaft verhindert, dass beim Einschieben in den Markraum eines Knochens, beispielsweise durch die zum freien Ende offenen Schlitze, weiteres Mark mitgenommen und zerstört wird. Zudem bietet das freie Ende des Spreizkörpers die Möglichkeit, als Röntgenmarker zu fungieren. Dadurch ist gewährleistet, dass über eine Röntgenaufnahme die exakte Position der an sich beispielsweise aus Kunststoff bestehenden Gelenkteilprothese lagegerecht kontrolliert werden kann.

Eine weitere mögliche Ausführungsform sieht vor, dass auf der Aussenwandung des Schaftes Anti-Verdreheinrichtungen vorgesehen sind. Diese Anti-Verdreheinrichtungen sind finnenartige Konstruktionen, die mindestens einmal auf dem Umfang angeordnet sind und sich in Längserstreckung des Schaftes ausdehnen. Sie sind derart ausgebildet, dass sie den Spreizvorgang der Spreizhülse entsprechend nicht behindern und zusätzlich die Verankerung des Implantats innerhalb des Markraumes unterstützen.

Eine bevorzugte Ausführungsform dieser Anti-Verdrehsicherung sieht vor, dass sie vom freien Ende ausgehend in Richtung der distalen oder proximalen Komponente in ihren Ausladungen zunehmen, so dass beim Einführen des Implantats eine Verdrehung noch möglich ist und je weiter das Implantat in den Markraum hineingeführt wird, desto eher wird die Verdrehung entsprechend eingeschränkt.

Um solche Anti-Verdreheinrichtungen zusammen mit dem Schaft einer Komponente installieren zu können, kann vorgesehen sein, den Hohlraum mit einem Räumwerkzeug durch drehende und nicht hämmernde Bewegungen rotationssymmetrisch zu räumen. Ein zusätzliches Hilfsmittel, wie beispielsweise eine Lehre, unterstützt auch das Räumen für die finnenartigen Ausgestaltungen, so dass das Implantat passgenau eingesetzt werden kann.

Die Gelenkteilprothese kann aus einem an sich aus dem Stand der Technik bekannten Material bestehen.

So ist es möglich, um insbesondere einen knochenähnlichen Elastizitätsmodul zu erreichen, dass hierfür ein entsprechender Kunststoff verwendet wird.

Dies bringt auch insbesondere den Vorteil mit sich, dass die Gelenkteilprothesen mittels eines Spritzgussverfahrens hergestellt werden können.

Weitere vorteilhafte Ausgestaltungen gehen aus der nachfolgenden Beschreibung, den Zeichnungen sowie den Ansprüchen hervor.

### Zeichnungen

Es zeigen:
- Fig. 1: eine menschliche Hand zur Verdeutlichung der Platzierung der Gelenkteilprothesen;
- Fig. 2: eine erste perspektivische Ansicht auf die erfindungsgemäße Gelenkteilprothese mit proximaler Komponente;
- Fig. 3: eine weitere Darstellung einer perspektivischen Ansicht auf die erfindungsgemäße Gelenkteilprothese mit distaler Komponente;
- Fig. 4: einen Schnitt durch die Gelenkteilprothese gemäss Fig. 2 entlang der Ebene IV-IV;
- Fig. 5: einen Schnitt durch die Gelenkteilprothese gemäss Fig. 3 entlang der Ebene V-V;
- Fig. 6: eine Seitenansicht auf einen in den Schaft der Gelenkteilprothese einsetzbaren Spreizkörper;
- Fig. 7: eine perspektivische Ansicht auf eine Gelenkteilprothese, jedoch gegenüber Fig. 2 und Fig. 3 im gespreizten Zustand;
- Fig. 8: eine erfindungsgemäße Gelenkteilprothese mit einer ersten Ausführung einer Anti-Verdrehsicherung;
- Fig. 9: eine erfindungsgemäße Gelenkteilprothese mit einer weiteren Ausführung einer Anti-Verdreheinrichtung.

### Beschreibung von Ausführungsbeispielen

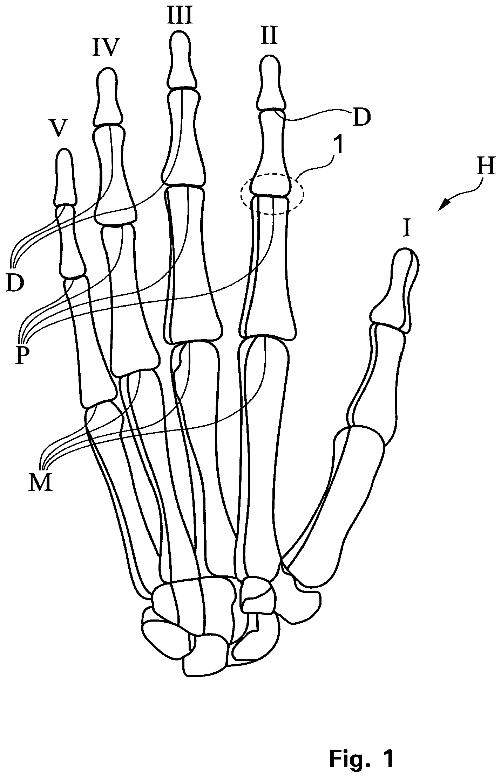

In Fig. 1 ist eine Hand H eines menschlichen Körpers in Knochenstruktur gezeigt. Um die Nomenklatur für die Anbringung von Gelenkteilprothesen, wie sie nachfolgend beschrieben werden, zu definieren, sind in Fig. 1 folgende Bezeichnungen angegeben: In Fig. 1 sind im Bereich der Fingerspitzen die distal interphalangealen Gelenke D angeordnet. Ihnen folgen die proximal interphalangealen Gelenke P sowie die im Mittelhandbereich angeordneten metacarporalen phalangealen Gelenke M. Die einzelnen Finger sind mit I bis V gekennzeichnet.

### Proximale Gelenkteilprothese:

Die nachfolgend beschriebenen Gelenkteilprothesen sind insbesondere in den distal als auch in den proximal interphalangealen Bereichen D, P aber auch in den metacarporalen phalangealen Gelenken M anwendbar. Sie sind unabhängig von der Fingerwahl I bis V. Sie unterscheiden sich lediglich in der Größe und in der jeweiligen Auslegung der proximalen oder distalen Komponenten.

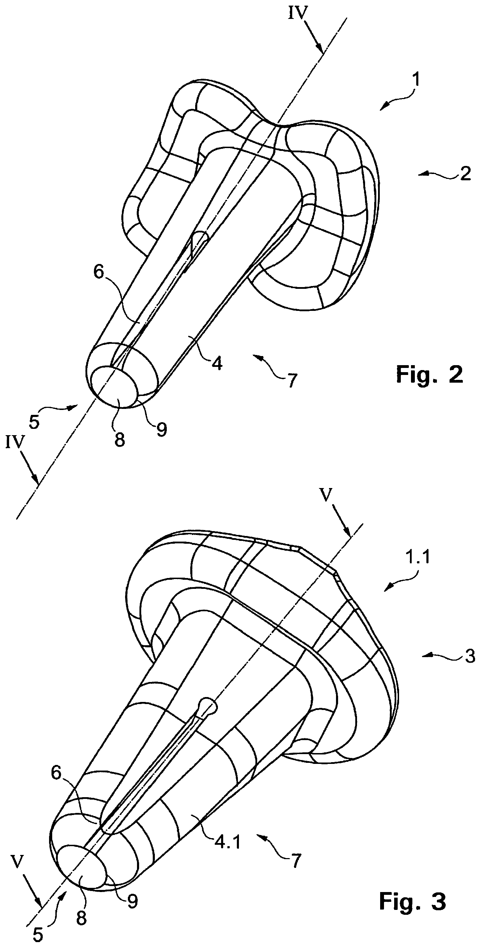

In Fig. 2 ist eine perspektivische Ansicht einer Gelenkteilprothese 1 mit einer proximalen Komponente 2 dargestellt. Die proximale Komponente 2 weist eine nicht bei dieser Ausführung näher dargestellte Gleitfläche auf, auf der eine in Fig. 3 dargestellte distale Komponente 3 einer Gelenkteilprothese 1.1 gleiten kann.

Von der proximalen Komponente 2 erstreckt sich rückwärtig ein Schaft 4, der sich nahezu senkrecht von der proximalen Komponente 2 absteht und bei dem hier dargestellten Ausführungsbeispiel eine Verjüngung zu seinem freien Ende 5 hin aufweist. Er ist im Querschnitt rund ausgestaltet.

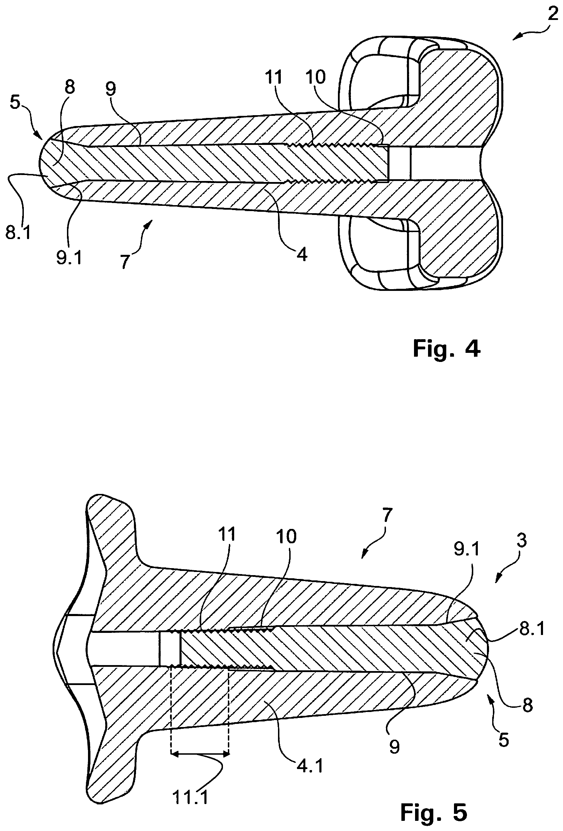

Das freie Ende 5 des Schaftes 4 weist ferner Schlitze 6 auf. Diese Schlitze 6 bilden eine Spreizhülse 7, die zumindest als Teil des Schaftes 4 ausgebildet ist. Innerhalb des Schaftes 4 bzw. der Spreizhülse 7 ist ein Spreizkörper 8 angeordnet. Der Spreizkörper 8 liegt in einer innerhalb der Spreizhülse 7 bzw. des Schaftes 4 vorgesehenen Bohrung 9. Wie in Fig. 4 dargestellt, erstreckt sich die Bohrung 9 von dem freien Ende 5 des Schaftes 4 bis hin über die proximale Komponente 2 der Gelenkteilprothese 1 hinweg. An dem freien Ende 5 des Schaftes 4 ist die Bohrung 9 im Vergleich zum übrigen Schaft 4 mit einem breiteren Bohrungsabschnitt 9.1 ausgebildet.

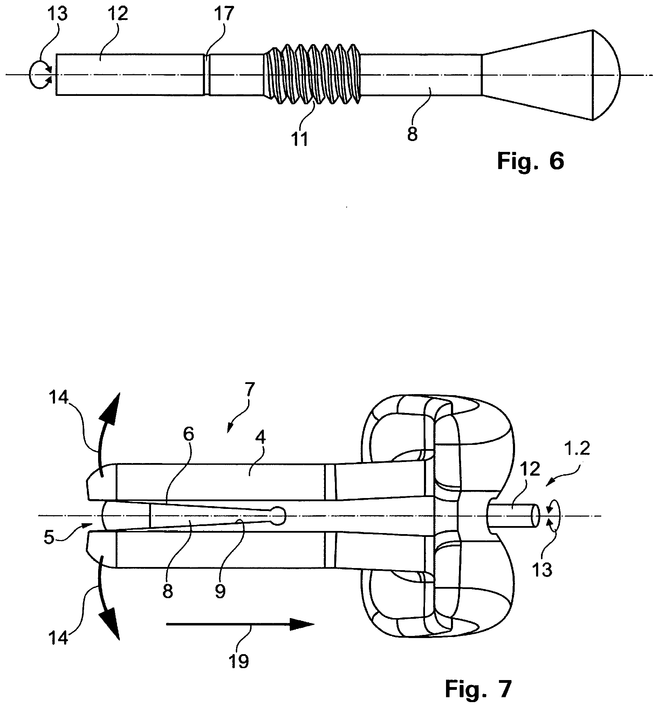

Die Bohrung 9 dient dazu, den entsprechenden Spreizkörper 8 aufzunehmen, so wie es in Fig. 6 dargestellt ist. Das freie Ende des Spreizkörpers 8 - bezeichnet mit 8.1- ist ebenfalls breiter ausgebildet, so dass bei Bewegungen des Spreizkörpers 8 von dem freien Ende 5 in Richtung der proximalen Komponente 2 die Spreizhülse 7 gespreizt wird.

Das freie Ende 8.1 des Spreizkörpers 8 ist so abgerundet ausgebildet, dass es leicht über das freie Ende 5 des Schaftes 4 absteht. Dadurch wird beim Einführen des Schaftes 4 in einen Markraum das übrige Mark geschützt, denn es muss vermieden werden, dass die scharfen Kanten der Schlitze 6 etwaiges Mark des Knochens verletzen.

Darüber hinaus ist die Wandung des Schaftes 4 insbesondere im Bereich des freien Endes 5 abgerundet ausgebildet.

### Distale Gelenkteilprothese:

In Fig. 3 ist eine perspektivische Ansicht einer Gelenkteilprothese 1.1 mit einer distalen Komponente 3 dargestellt. Die distale Komponente 3 weist eine nicht bei dieser Ausführung näher dargestellte Gleitfläche auf, auf der die in Fig. 2 dargestellte proximale Komponente 2 gleiten kann.

Von der distalen Komponente 3 erstreckt sich rückwärtig ein Schaft 4.1, der sich nahezu senkrecht von der distalen Komponente 3 aus erstreckt und bei dem hier dargestellten Ausführungsbeispiel eine Verjüngung zu seinem freien Ende 5 hin aufweist. Er ist im Querschnitt rund ausgestaltet, so dass eine Anpassung an den Markraum des Knochens möglich ist.

Das freie Ende 5 des Schaftes 4.1 weist ferner Schlitze 6 auf. Diese Schlitze 6 bilden eine Spreizhülse 7, die zumindest als Teil des Schaftes 4.1 ausgebildet ist. Innerhalb der Spreizhülse 7 ist ein Spreizkörper 8 angeordnet, der sich innerhalb der Spreizhülse 7 bzw. des Schaftes 4.1 vorgesehenen Bohrung 9 befindet. Wie in Fig. 5 dargestellt, erstreckt sich die Bohrung 9 von dem freien Ende 5 des Schaftes 4.1 bis hin über die distale Komponente 3 der Gelenkteilprothese 1.1 hinweg. An dem freien Ende 5 des Schaftes 4 ist die Bohrung 9 in einem Abschnitt 9.1 breiter ausgebildet.

Die Bohrung 9 dient dazu, den entsprechenden Spreizkörper 8 aufzunehmen, so wie es in Fig. 6 dargestellt ist. Das freie Ende 8.1 des Spreizkörpers 8 ist ebenfalls breiter ausgebildet, so dass bei Bewegungen des Spreizkörpers 8 von dem freien Ende 5 in Richtung der distalen Komponente 3 die Spreizhülse 7 gespreizt wird.

Auch hier ist bei dem Spreizkörpers 8 vorgesehen, dass dieser leicht über das freie Ende 5 absteht, sodass beim Einführen des Schaftes 4.1 in einen Markraum das übrige Mark geschützt ist, da vermieden wird, dass die scharfen Kanten der Schlitze 6 etwaiges Mark verletzen.

Auch hier ist die Wandung des Schaftes 4.1 im Bereich des freien Endes 5 abgerundet ausgebildet, und zwar um vermeiden, dass beim Einschieben der Gelenkteilprothese 1.1 ungewollte Verletzungen des Gewebes auftreten.

Innerhalb der Bohrung 9 ist im Bereich der Schäfte 4 und 4.1 ein Gewinde 10 vorgesehen, das mit einem in der Fig. 6 dargestellten Gewinde 11 des Spreizkörpers 8 zusammenwirkt. Da der Spreizkörper 8 zumindest im Anfangsbereich ebenfalls das Gewinde 11 aufweist, wird bei dessen Einführung in die Bohrung 9 und durch ein Drehen des Spreizkörpers 8 zusätzlich (neben dem Zusammenwirken mit den Gewinde 10 innerhalb der Bohrung 9) das Gewinde 11 in den Schaft 4 bzw. 4.1, der vorzugsweise aus Kunststoff besteht, eingeschnitten, was durch den Pfeil 11.1 in Fig. 5 angedeutet ist.. Dadurch wird gewährleistet, dass der Spreizkörper 8 aufgrund der Haftreibung seine Position beibehält und damit auch die Spreizung aufrechterhalten bleibt, die nach dem Eindrehen des Spreizkörpers 8 durchgeführt worden ist.

Der Spreizkörper 8 in der in Fig. 6 dargestellten Ausführung weist eine Sollbruchstelle 17 auf. Von der Sollbruchstelle 17 weg erstreckt sich in Gegenrichtung des vorgesehenen freien Endes 8.1 ein Hilfsmittel 12, das zum Verdrehen des Spreizkörpers 8 in und in dessen Gegenrichtung vorgesehen ist.

In Fig. 7 ist eine Gelenkteilprothese 1.2 in perspektivischer Ansicht gezeigt, die abweichend von ihrer Ausgangsstellung entsprechend wie sie in den Fig. 2 und 3 dargestellt ist, eine Spreizstellung einnimmt.

Um den Spreizkörper 8 nun in einer Pfeilrichtung 13 bewegen zu können, damit eine Spreizung der Spreizhülse 7 erfolgt, ist an den Spreizkörper 8 das Hilfsmittel 12 (auch in Fig. 6 dargestellt) vorgesehen, das mit dem Spreizkörper 8 über die Sollbruchstelle 17 zusammenwirkt. Die Zusammenwirkung ist derart ausgebildet, dass durch ein Drehen in Pfeilrichtung 13 der Spreizkörper 8 in eine Pfeilrichtung 19 bewegt wird und sich über die Gewindepaarung 10/11 (in Fig. 4 bis 6 dargestellt) sich in Pfeilrichtung 19 verfährt und dabei teilweise das Gewinde 10 auch in die Gelenkteilprothese 1.2 einschneidet, und zwar wie es in Fig. 5 angedeutet ist. Mit zunehmender Spreizung (Pfeile 14) nimmt das aufzubringende Drehmoment auf das Hilfsmittel 12 zu, so dass bei einer definierten Größe das Hilfsmittel 12 sich von dem übrigen Spreizkörper 8 vorzugsweise an der in Fig. 6 beschriebenen Sollbruchstelle 17 trennt. Dieses Hilfsmittel 12 kann dann entnommen werden, ohne dass entsprechende Späne im Bereich der Gelenkteilprothese 1.2 entstehen bzw. zurückbleiben. Der Sollbruch findet dabei innerhalb der Gelenkteilprothese 1.2 statt, so dass die Gleitflächen weiterhin ihre gewünschte Eigenschaft beibehalten. Die Gelenkteilprothese 1.2 ist nun im Markraum fixiert und durch Spreizung der Spreizhülse 7 innerhalb eines Knochenmarkraums zumindest in Pfeilrichtung 19 fixiert.

Als Alternative kann auch vorgesehen werden, dass anstelle des Hilfsmittels 12 ein Werkzeug eingeführt wird, das beispielsweise über einen Innensechskantschrauber mit dem Spreizkörper 8 zu koppeln ist.

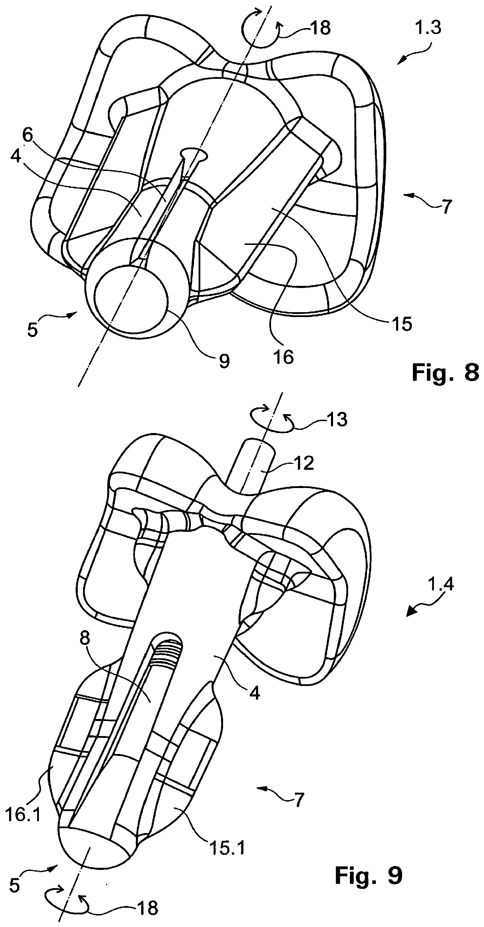

In Fig. 8 ist eine weitere Möglichkeit gezeigt, wie die erfindungsgemäßen Ausführungsformen verbessert werden können: Die dort dargestellte Gelenkteilprothese 1.3 weist neben den zuvor bereits beschriebenen Eigenschaften zusätzlich ein Antiverdrehelement 15 auf. Dieses Anti-Verdrehelement 15 dient dazu, eine einmal in einem Markraum, der vorzugsweise zumindest nahezu den Querschnitt einnimmt wie der Schaft 4 und das Antiverdrehelement 15 zusammen, eingeführte Gelenkteilprothese 1.3 zu fixieren. Dies geschieht dabei derart, dass beim Verdrehen des Spreizkörpers 8 in oder gegen die Pfeilrichtung 18 ein Verdrehen der Gelenkteilprothese 1.3 innerhalb des Markraumes nicht mehr möglich ist. Das Anti-Verdrehelement 15 besitzt in Richtung des Schaftes 4 finnenartige Ausbildungen 16 , die sich entlang des Schaftes 4 erstrecken. Insbesondere im Bereich des freien Endes 5 des Schaftes 4 ist vorgesehen, die Anti-Verdreheinrichtung 15 sehr geringfügig auszubilden, so dass beim Einführen noch ein entsprechendes Positionieren innerhalb des Markraumes möglich ist. Je tiefer die Gelenkteilprothese 1.3 in den Markraum eingeführt wird, desto mehr durchdringen die finnenartigen Ausbildungen 16 der Anti-Verdreheinrichtungen 15, die mindestens mit einem Teil auf dem Umfang des Schaftes 4 angeordnet ist, den Markraum und schneiden und drücken sich in das Mark des Knochens ein, wodurch eine entsprechende Keilwirkung entsteht.

Eine weitere alternative Ausführung in der Form einer Gelenkteilprothese 1.4 ist in Fig. 9 dargestellt. Diese Ausführung unterscheidet sich von der Ausführung gemäss Fig. 8 dadurch, dass die finnenartigen Ausbildungen -bezeichnet mit 16.1- des hier vorliegenden Anti-Verdrehelements 15.1 sich nur teilweise über die Längserstreckung des Schaftes 4 erstrecken.

Die Anti-Verdrehelemente 15 und 15.1 verhindern rotatorische Bewegungen der Gelenkteilprothesen 1.3 und 1.4 in oder gegen die Pfeilrichtung 18. Sie dient zumindest zusätzlich dazu, ein Fixieren der Gelenkteilprothese 1.3 und 1.4 in deren jeweiliger Längserstreckung zu erwirken.

Sämtliche aufgezeigten Gelenkteilprothesen 1 - 1.4 zeigen somit die Eigenschaft, dass durch Ausbildung des Schaftes 4 zusammen mit der zumindest teilweisen Spreizhülse 7 eine dübelartige Ausbildung des Schaftes 4 bereitgestellt wird, die bewirkt, dass die Gelenkteilprothesen 1 -1.4, die in einen Markraum des Knochens eingeführt werden, in Einführungsrichtung beziehungsweise in dessen Gegenrichtung fixiert sind. Ein Lösen der Gelenkteilprothesen 1 -1.4, hervorgerufen durch mechanische Bewegungen aber auch durch Verringerung des Markes im Markraum des Knochens oder durch sonstige chemische, biologische oder physikalische Eigenschaften, wird somit umgangen. Zudem ist der Schaft 4 rau ausgebildet und weist mehrere Öffnungen auf, in die das entsprechende Mark hineinwachsen kann, so dass eine form- und kraftschlüssige Verbindung der Gelenkteilprothese 1 mit dem übrigen Teil des Knochens entstehen kann. Das Anti-Verdrehelement 15 unterstützt die positionsgerechte Ausrichtung der Gelenkteilprothese 1 und verhindert die Verdrehung in oder gegen die Pfeilrichtung 18. Durch die erfindungsgemäße Gelenkteilprothese 1 wird eine insbesondere gelenkschonende operative Anpassung und Positionierung einer Gelenkteilprothese 1 möglich.

### Bezugszeichenliste

- D: distale interphalangeale Gelenke
- P: proximale interphalangele Gelenke
- M: metacarporalen phalangeale Gelenke

- 1 -1.4: Gelenkteilprothese
- 2: Komponente (praximale)
- 3: Komponente (distale)
- 4; 4.1: Schaft
- 5: freies Schaftende
- 6: Schlitz
- 7: Spreizhülse
- 8: Spreizkörper
- 8.1: freies Ende (Spreizkörper)
- 9: Bohrung
- 9.1: breiter Bohrungsabschnitt
- 10: Gewinde (innen)
- 11: Gewinde (außen)
- 12: Hilfsmittel
- 13: Pfeilrichtung
- 14: Pfeile
- 15; 15.1: Anti-Verdrehelement
- 16: finnenartige Ausbildungen
- 17: Sollbruchstelle
- 18: Pfeilrichtung
- 19: Pfeilrichtung

## Patentansprüche

1. Gelenkteilprothese, insbesondere für ein Fingergelenk, bestehend entweder aus einer proximalen oder distalen Komponente sowie einem von dieser Komponente wegweisenden, in einem Knochen zu lagernden Schaft, der zumindest zum Teil als eine Spreizhülse ausgebildet ist und eine durchgehende Bohrung aufweist, innerhalb der ein in dieser verschiebbarer Spreizkörper vorgesehen ist, **dadurch gekennzeichnet, dass** zumindest jeweils im Anfangsbereich der durchgehenden Bohrung (9) des mit der Spreizhülse (7) ausgebildeten Schaftes (4) und des Spreizkörpers (8) ein Gewinde (10; 11) vorgesehen ist, welche derart zusammenwirken, dass nach einem Eindrehen des Spreizkörpers (8) in die Bohrung (9), neben dem Zusammenwirken der beiden Gewinde (10; 11), teilweise das Gewinde (11) des Spreizkörpers (8) zur Erzeugung einer Haftreibung zusätzlich in die Bohrung (9) des Schaftes (4) eingeschnitten (11.1) ist.

2. Gelenkteilprothese nach Anspruch 1, deren Spreizhülse endseitig Schlitze aufweist, **dadurch gekennzeichnet, dass** die Bohrung (9) des Schaftes (4; 4.1) im Bereich der Spreizhülse (7) an deren freiem Ende (5) - Bohrungsabschnitt 9.1- und das freie Ende (8.1) des Spreizkörpers (8) jeweils breiter ausgebildet ist.

3. Gelenkteilprothese nach Anspruch 2, **dadurch gekennzeichnet, dass** das freie Ende (5) des Schaftes (4; 4.1) und das freie Ende (8.1) des Spreizkörpers (8) zusammen leicht abstehend gerundet gestaltet sind.

4. Gelenkteilprothese nach Anspruch 3, **dadurch gekennzeichnet, dass** im nicht gespreizten Zustand des Schaftes (4) das freie Ende (5) des Schaftes (4) zusammen mit dem freien Ende (8.1) des Spreizkörpers (8)eine geschlossene Einheit bilden.

5. Gelenkteilprothese nach mindestens einem der vorhergehenden Ansprüche, **dadurch gekennzeichnet, dass** der Schaft (4; 4.1) und/oder der Spreizkörper (8) aus Kunststoff bestehen.

6. Gelenkteilprothese nach mindestens einem der vorhergehenden Ansprüche, **dadurch gekennzeichnet, dass** der Spreizkörper (8) für dessen Bewegung in und gegen die Drehrichtung (Pfeilrichtung 13), vom Bereich der Komponente (2; 3) aus, hierfür in der Bohrung (9) des Schaftes (4; 4.1) ein Hilfsmittel (12) aufweist.

7. Gelenkteilprothese nach Anspruch 6, **dadurch gekennzeichnet, dass** das Hilfsmittel (12) über eine Sollbruchstelle (17) mit dem Spreizkörper (8) einteilig verbunden ist.

8. Gelenkteilprothese nach Anspruch 7, **dadurch gekennzeichnet, dass** die Sollbruchstelle (17) derart ausgelegt ist, dass sich das Hilfsmittel (12) bei einer definierten Größe eines Drehmomentes von dem Spreizkörper (8) trennt.

9. Gelenkteilprothese nach mindestens einem der vorhergehenden Ansprüche, **dadurch gekennzeichnet, dass** der Spreizkörper (8) einen Röntgenmarker aufweist.

10. Gelenkteilprothese nach mindestens einem der vorhergehenden Ansprüche, **dadurch gekennzeichnet, dass** der Schaft (4; 4.1) eine raue Oberfläche aufweist.

## Claims

1. Prosthetic joint part, in particular for a finger joint, consisting of either a proximal or distal component, and also of a shaft which extends away from this component, is to be mounted in a bone, is configured at least in part as an expansion sleeve and has a continuous bore within which an expansion body is provided that is displaceable therein, **characterized in that** respective threads (10; 11) are provided at least in the starting region of the continuous bore (9) of the shaft (4) formed with the expansion sleeve (7), and of the expansion body (8), which threads (10; 11) interact in such a way that, after the expansion body (8) has been screwed into the bore (9), not only do the two threads (10; 11) interact, the thread (11) of the expansion body (8) is in addition partially cut (11.1) into the bore (9) of the shaft (4) in order to generate static friction.

2. Prosthetic joint part according to Claim 1, the expansion sleeve of which having slits at the end, **characterized in that** the bore (9) of the shaft (4; 4.1), in the region of the expansion sleeve (7) at the free end (5) thereof - bore portion 9.1, - and the free end (8.1) of the expansion body (8) are each broader.

3. Prosthetic joint part according to Claim 2, **characterized in that** the free end (5) of the shaft (4; 4.1) and the free end (8.1) of the expansion body (8) together form a slightly protruding rounded shape.

4. Prosthetic joint part according to Claim 3, **characterized in that**, in the unexpanded state of the shaft (4), the free end (5) of the shaft (4) and the free end (8.1) of the expansion body (8) form a closed unit.

5. Prosthetic joint part according to at least one of the preceding claims, **characterized in that** the shaft (4; 4.1) and/or the expansion body (8) are made of plastic.

6. Prosthetic joint part according to at least one of the preceding claims, **characterized in that**, for its movement in and counter to the rotation direction (arrow direction 13), the expansion body (8) has, starting from the region of the component (2; 3), an auxiliary means (12) provided for this purpose in the bore (9) of the shaft (4; 4.1).

7. Prosthetic joint part according to Claim 6, **characterized in that** the auxiliary means (12) is connected in one piece to the expansion body (8) via a predetermined breaking point (17).

8. Prosthetic joint part according to Claim 7, **characterized in that** the predetermined breaking point (17) is configured in such a way that the auxiliary means (12) separates from the expansion body (8) at a defined extent of a torque.

9. Prosthetic joint part according to at least one of the preceding claims, **characterized in that** the expansion body (8) has an X-ray marker.

10. Prosthetic joint part according to at least one of the preceding claims, **characterized in that** the shaft (4; 4.1) has a rough surface.

## Revendications

1. Prothèse de partie d'articulation, en particulier pour une articulation de doigt, constituée soit d'un composant proximal soit d'un composant distal ainsi que d'une tige opposée à celui-ci et destinée à être logée dans un os, laquelle est réalisée au moins en partie sous forme de douille expansible et comprend un alésage traversant à l'intérieur duquel est prévu un corps expansible déplaçable dans celui-ci, **caractérisée en ce qu'**un filetage (10 ; 11) est prévu respectivement au moins dans la région de départ de l'alésage traversant (9) de la tige (4) comprenant la douille expansible (7) et du corps expansible (8), lesquels filetages coopèrent de telle sorte que suite à un vissage du corps expansible (8) dans l'alésage (9), en plus de la coopération des deux filetages (10 ; 11), le filetage (11) du corps expansible (8) entaille (11.1) en outre partiellement l'alésage (9) de la tige (4) pour produire un frottement par adhérence.

2. Prothèse de partie d'articulation selon la revendication 1, dont la douille expansible comprend des fentes côté extrémité, **caractérisée en ce que** l'alésage (9) de la tige (4 ; 4.1) dans la région de la douille expansible (7) à son extrémité libre (5) - partie d'alésage (9.1) - et l'extrémité libre (8.1) du corps expansible (8) sont formés respectivement de manière plus large.

3. Prothèse de partie d'articulation selon la revendication 2, **caractérisée en ce que** l'extrémité libre (5) de la tige (4 ; 4.1) et l'extrémité libre (8.1) du corps expansible (8) sont configurées conjointement de manière arrondie de façon légèrement saillante.

4. Prothèse de partie d'articulation selon la revendication 3, **caractérisée en ce qu'**à l'état non écarté de la tige (4), l'extrémité libre (5) de la tige (4) forme, conjointement avec l'extrémité libre (8.1) du corps expansible (8), une unité fermée.

5. Prothèse de partie d'articulation selon au moins l'une des revendications précédentes, **caractérisée en ce que** la tige (4 ; 4.1) et/ou le corps expansible (8) sont constitués de matière synthétique.

6. Prothèse de partie d'articulation selon au moins l'une des revendications précédentes, **caractérisée en ce que** le corps expansible (8) comprend, pour son déplacement dans le sens de rotation (flèches 13) et en sens inverse à celui-ci, à partir de la région du composant (2 ; 3), un moyen auxiliaire (12) à cet effet dans l'alésage (9) de la tige (4 ; 4.1).

7. Prothèse de partie d'articulation selon la revendication 6, **caractérisée en ce que** le moyen auxiliaire (12) est relié au corps expansible (8) d'un seul tenant par le biais d'un point destiné à la rupture (17).

8. Prothèse de partie d'articulation selon la revendication 7, **caractérisée en ce que** le point destiné à la rupture (17) est configuré de telle sorte que le moyen auxiliaire (12) se sépare du corps expansible (8) en cas d'ampleur définie d'un couple.

9. Prothèse de partie d'articulation selon au moins l'une des revendications précédentes, **caractérisée en ce que** le corps expansible (8) comprend un marqueur radiographique.

10. Prothèse de partie d'articulation selon au moins l'une des revendications précédentes, **caractérisée en ce que** la tige (4 ; 4.1) présente une surface rugueuse.
